# EUROPEAN PATENT APPLICATION

(11) **EP 3 928 667 A1**
(43) Date of publication of application: **29.12.2021**
(21) Application number: 21181863.8
(22) Date of filing: 25.06.2021
(51) Int. Cl.: A47J 31/52

(54) **METHOD OF MONITORING AT LEAST ONE CHARACTERISTIC OF A TYPE OF COFFEE**

(30) Priority: 26.06.2020 IT 202000015532
(71) Applicant: Gruppo Cimbali S.p.A., 20082 Binasco (MI) (IT)
(72) Inventor: Alberto, Galimberti, 20082 Binasco MI (IT); Volonté, Claudio, 20082 Binasco MI (IT)
(74) Representative: Perani & Partners S.p.A.

(57) **Abstract**

A method of monitoring at least one characteristic of a type of coffee from a plurality of reference characteristics associated with each type of coffee, each reference characteristic being associated with a respective reference signal, said method comprising the steps of:
- sensing, by one or more sensors (301), the concentration of one or more volatile organic compounds in a mixture of volatile organic compounds released from the coffee in a sampling time interval;
- generating a coffee-representative sampling signal for each sampling time interval, as a function of the volatile organic compounds;
- comparing each sampling signal so generated to detect a change over time in one or more volatile organic compounds released from the coffee at successive sampling time intervals;
- generating a signal representative of the change over time in one or more volatile organic compounds released from the coffee when the change is sensed;
- comparing the change-representative signal so generated with the reference signals associated with each reference characteristic;
- generating a characteristic-recognition signal as a function of the comparison between the signal representative of the change over time and the reference signals.

## Description

### Technical field

The present invention relates to a method of monitoring at least one characteristic of a type of coffee preferably stored in a hopper of a grinder or grinder-doser. The present invention also relates to an apparatus and a system for monitoring at least one characteristic of a type of coffee.

### Background art

The equipment required for the preparation of the coffee cups, i.e. the grinder and the coffee machine, must be suitably regulated. Coffee beans have peculiar characteristics that should be accounted for when calibrating and programming the equipment. Namely, account must be taken of the blend in use, the degree of roast of the blend or the date of packaging of the coffee in the package and also of the change of given features of the coffee during storage in the package, the grinder or the coffee machine. For these reasons, each type of coffee in use must be suitably ground by a specific grinder-doser and treated with pressurized hot water with given pressure and temperature values according to the type itself and according to its characteristics.

In particular, in order to prevent the hoppers of grinders associated with a certain type of coffee with given characteristics from grinding coffee in a wrong manner, various solutions have been developed. For example, known solutions are detecting the concentration of Total Volatile Organic Compounds (TVOC) and acting on the grinding parameters if a concentration change exceeding preset parameters is recorded.

### Problems of the prior art

Prior art devices cannot discriminate a change of a given characteristic of coffee and cannot properly change preparation parameters known as grinding parameters (particle size and dose of coffee) and brewing parameters (pressure and temperature of hot water). In particular, prior art devices change the preparation parameters in response to overall data expressing changes in concentrations of volatile organic compounds without accounting for any interaction between the volatile organic compounds associated with one or more characteristics of coffee. As a result, prior art devices lead to a product with different organoleptic properties being dispensed into the cup.

### Object of the invention

The object of the present invention is to provide a method, an apparatus and a system that can obviate the above discussed drawbacks of the prior art.

In particular, the object of the present invention is to provide a method that can discriminate different characteristics of each type of coffee and afford proper changing of preparation parameters as well as warn the operator of any errors during coffee loading.

The aforementioned technical purpose and objects are substantially fulfilled by a method of monitoring at least one characteristic of each type of coffee, an apparatus and a system that comprise the technical features as disclosed in one or more of the accompanying claims.

### Advantages of the Invention

Advantageously, the method, the apparatus and the system of the present invention can detect of one or more specific volatile organic compounds released from the coffee stored in the hopper.

Advantageously, the method, the apparatus and the system of the present invention can recognize the change of at least one characteristic of the coffee stored in the hopper from a plurality reference characteristics

Advantageously, the method, the apparatus and the system of the present invention can properly change coffee preparation parameters while accounting for each recognized characteristic.

Advantageously, the method, the apparatus and the system of the present invention can warn the bartender or operator if the hopper contains a coffee having one or more characteristics other than the predetermined characteristics.

### BRIEF DESCRIPTION OF FIGURES

Further features and advantages of the present invention will result more clearly from the illustrative, non-limiting description of a preferred, non-exclusive embodiment of a method, a machine and a system for monitoring a characteristic of each type of coffee as shown in the accompanying drawings:

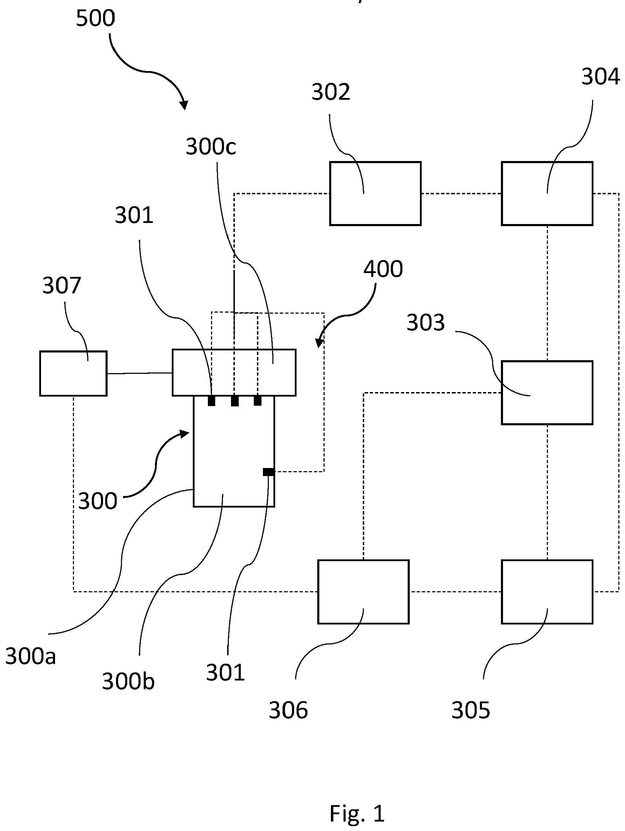

- Figure 1 shows a diagrammatic view of the machine for monitoring a characteristic of the coffee according to the present invention;
- Figure 2 shows a curve of the electrical resistance of a sensor as used in the method, the apparatus and the system of the present invention;
- Figure 3 shows the graph of the first derivative of Figure 2;

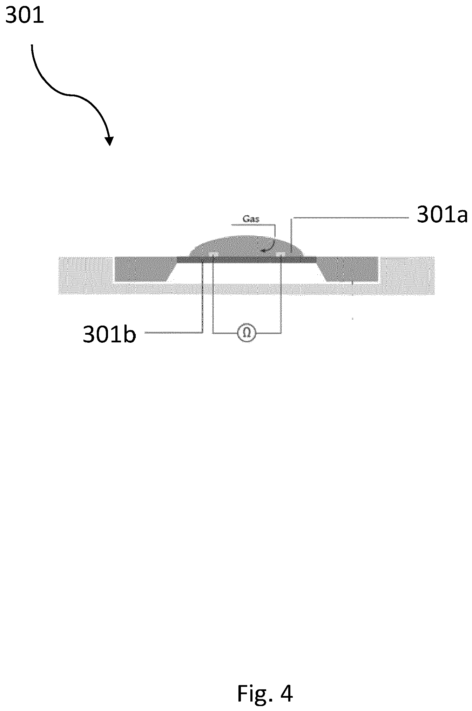

- Figure 4 shows a diagrammatic view of a sensor of the present invention.

### DETAILED DESCRIPTION

Even when not expressly stated, the individual features as described with reference to the particular embodiments shall be intended as auxiliary to and/or interchangeable with other features described with reference to other exemplary embodiments.

The present invention relates to a method of monitoring at least one characteristic a type of coffee from a plurality of reference characteristics having a reference signal associated therewith. Preferably, the characteristic to be recognized is associated with the coffee contained in a hopper 300 associated with a grinder or a grinder-doser or an espresso machine. In particular, the hopper 300 is part of a coffee grinding and/or dosing device.

The method comprises the steps as set forth hereinbelow, performed according to a preferred embodiment.

The method of monitoring at least one characteristic of coffee comprises the step of sensing, by one or more sensors 301, the concentration of one or more volatile organic compounds in a mixture of volatile organic compounds released from the coffee, preferably held within the hopper 300 in a sampling time interval. This step of sensing is periodically carried out at successive sampling time intervals and/or as directed by a user and/or alternatively as directed by a control unit,.

According to a preferred embodiment, the step of sensing is carried out at predefined sampling time intervals to detect the change in the concentrations of one or more volatile organic compounds. Preferably, each time interval ranges from half an hour to 4 hours, more preferably comprised from one hour to three hours, according to the preferred embodiment the time interval is 2 hours.

In particular, it shall be noted that the volatile organic compounds released from the coffee in a container, preferably a hopper, are characteristic of the type of coffee, either single-origin or blend and the residence time inside the container and any manufacturing defects. In detail, each type of coffee releases a plurality of volatile organic compounds in different concentrations, that can be recognized, for example, by means of gas chromatography-mass spectrometry. More in detail, the characteristics of each type of coffee are characterized by a given combination of concentrations of volatile organic compounds and may vary over time, such as the molecule that is responsible for rancidity which increases its concentration or the floral molecule that reduces its concentration over time. Furthermore, certain characteristics of a type of coffee may have different concentrations because coffee, even of the same type, has been processed, transported and stored in a different manner. This may happen if one type of coffee is loaded into the hopper from different batches.

Advantageously, the method of the invention can monitor the concentrations of the volatile organic compounds associated with one or more characteristics of one type of coffee at sampling time intervals.

It should be noted that each sensor 301 is configured to record the concentration of certain specific organic compounds. More preferably, the sensors 301 are configured to sense the concentration of at least the following volatile organic compounds: pyrazine derivatives, furan derivatives, carboxylic acids, pyrrole derivatives, furfural derivatives and pyridine derivatives. This list of volatile organic compounds is characteristic of the various characteristics of the types of coffee. In particular, the discrimination between characteristics is associated with the concentration of the above listed volatile organic compounds.

According to a preferred embodiment, the step of detecting, by one or more sensors 301, the concentration of one or more volatile organic compounds comprises the sub-step of sending at least each detected concentration to a data processing unit.

The method of monitoring at least one characteristic of a type of coffee comprises the step of generating a sampling signal representative of the coffee contained in the hopper 300 according to the concentrations of the volatile organic compounds that have been sensed. Preferably, the method includes generating a sampling signal for each sampling time interval in which the concentrations have been sensed. Thus, the concentrations of volatile organic compounds and the characteristics associated therewith may be monitored over time.

In particular, the step of generating the sampling signal is carried out by the data processing unit 303 upon reception of the data sent from each sensor 301.

According to a preferred embodiment, the step of generating the sampling signal generates a signal representative of the concentrations of volatile organic compounds released from the coffee in a sampling time interval inside the hopper 300.

The method of monitoring a characteristic of a type of coffee comprises the step of comparing each sampling signal so generated to detect a change over time in one or more volatile organic compounds released from the coffee at successive sampling time intervals with a reference signal associated with each type of reference coffee. In particular, the step of comparing the sampling signals senses a change over time in one or more concentrations of volatile organic compounds sensed in the sampling time intervals inside the hopper. Preferably, a change in concentrations over time is sensed if it exceeds a preset threshold value.

Advantageously, the step of comparing the sampling signals can identify which concentrations of organic compounds have changed between one sampling time interval and the next.

The method of monitoring a characteristic of a type of coffee comprises the step of generating a signal representative of the change over time in one or more volatile organic compounds released from the coffee when the change is sensed.

Preferably, the generation of a signal representative of a change over time generates a signal representative of the change over time in one or more concentrations of volatile organic compounds sensed inside the hopper 300. More in detail, the signal representative of the change over time is associated with the concentration of one or more volatile organic compounds released from one respective reference type of coffee in different conditions.

The method of monitoring a characteristic a type of coffee comprises the step of comparing the change-representative signal with the reference signals associated with each reference characteristic. Preferably, the step of comparing the signal representative of the change over timed with the reference signals associated with each reference characteristic comprises the sub-step of recognizing the volatile organic compounds whose concentration has changed over time as a function of the change-representative signal. More preferably, the step of comparing the signal representative of the change over time and the reference signals comprises the sub-step of associating at least one reference characteristic with the recognized volatile organic compounds as a function of the reference signals.

The method of monitoring a characteristic of a type of coffee comprises the step of generating a characteristic-recognizing signal in response to the comparison of the generated representative signal with the reference signals.

According to a preferred embodiment, the step of generating a sampling signal and the step of generating a signal representative of a change over time, the step of comparing each sampling signal and the step of comparing the signal representative of a change over time with a reference signal are carried out using a neural network-based algorithm. Finally, the neural networks compare this change-representative signal generated with each reference signal so as to recognize the reference characteristic.

The neural network-based algorithm, which preferably resides in the data processing unit 303, undergoes a training process. In particular, the algorithm is trained to recognize a plurality of coffee characteristics by associating each of them with the concentration of one or more volatile organic compounds released from respective reference types of coffee. In detail, the neural network algorithm is trained to recognize one or more given characteristics of a type of coffee as a function of the concentration of certain organic compounds being released. Moreover, each characteristic of the type of coffee is associated with given grinding parameters (particle size and dose of coffee) and infusion parameters (pressure and temperature of hot water) to correct them if a change is sensed.

It shall be noted that the neural network algorithm defines control ranges according to the reference types of coffee used during the training process. Thus, the neural network recognizes the characteristic of the coffee even if the generated signal for the coffee in the hopper does not match the reference signal, because it falls within a control range predetermined and/or defined by the neural network.

Preferably, the neural network algorithm may be periodically updated by training it with additional reference characteristics. According to a preferred embodiment of the present invention, the plurality of reference characteristics comprises, for example, the rancid determined with the presence of any molecules of butyric acid and propanoic acid.

More preferably, the reference characteristics are the characteristics known to those of ordinary skill as associated with a single-origin coffee and fresh coffee blends and at different stages of residence inside a hopper.

The method of recognizing a type of coffee comprises the step of generating a recognition signal in response to the comparison of the signal representative of the change over time generated with the reference signals. In particular, the recognition signal carries the information concerning the changed characteristic of the coffee in the hopper. Thus, as the changed characteristics of the coffee are recognized, it is possible to act accordingly.

The method of recognizing a type of coffee comprises the step of generating a signal for indicating the change of at least one characteristic when a change of the concentrations between a sampling time interval and the next is sensed as a function of the control intervals of the neural network and of generating a signal for indicating that the preparation is continuing when a change in concentrations between one sampling time interval and the next is not sensed as a function of the control intervals of the neural network.

In particular, the step of generating a change-indicating signal and a continued preparation-indication signal comprises a sub-step of sending the change-indicating signal, preferably indicating the changed characteristic, or the continued preparation signal to a user interface 305 via a second data sending and receiving unit 304. The user interface 305 preferably comprises an application, residing in a mobile or fixed device and/or a display associated with the hopper 300.

The step of generating a change-indicating signal and the continued preparation-indicating signal comprises a sub-step of processing a text message associated with the change-indicating and the continued preparation-indicating signals respectively. This message informs the user of a possible automatic change of the grinding parameters and brewing parameters.

It shall be noted that the message can be customized by a user with the conventional steps known to the skilled person.

The step of generating a change-indicating signal and a continued-preparation indicating signal further comprises the sub-step of displaying the text message processed on the user interface 305. In a practical example, the processing unit 303 is configured to send the message to the user interface 305 as follows:
- via SMS and/or email;
- as a pop-up on the display associated with the hopper 300.

Advantageously, a message associated with the change-indicating signal will be displayed to warn the user of the change of a characteristic of the coffee in the hopper 300.

The method of monitoring at least one characteristic of coffee comprises a step of controlling a grinder, a grinder-doser or an espresso machine, preferably coupled to the hopper 300. In particular, the step of controlling is carried out by a control unit 306 associated with a grinder, a grinder-doser or an espresso machine. This control unit, 306, which is in signal communication with the data processing unit 303 and/or with the second data sending and receiving unit 304, is configured to enable, disable and control the parameters of a grinder, a grinder-doser or an espresso machine, preferably the grinding and brewing parameters. In detail, the step of controlling comprises the sub-steps of:
- receiving the recognition signal;
- generating a signal indicating the change of the grinding parameters and the brewing parameters for the coffee inside the hopper 300.

It shall be noted that the change of the grinding and brewing parameters is associated with the characteristic that has changed. Preferably, the monitoring method, once the change of concentrations has been sensed and the changed characteristic has been recognized, will change the grinding and brewing parameters according to the characteristic itself. More preferably, the monitoring method identifies the change of several characteristics and changes the grinding and brewing parameters according to the characteristics that have been identified.

According to a preferred embodiment, the method of monitoring at least one characteristic of a type of coffee comprises a step of confirming, by the user, to proceed with the changes of the grinding and brewing parameters. Thus, once the message of a possible automatic change of the grinding and brewing parameters has been received, the method includes confirming, by the user, the change of the grinding parameters. If the user refuses to change the grinding and brewing parameters, the control unit 306 will maintain the previous grinding and brewing parameters. It should be noted that the method also includes the possibility of resetting the grinding and brewing parameters to any previous configuration of the grinding parameters.

According to one embodiment, the method of monitoring at least one characteristic of a type of coffee may be used as part of a method of controlling the operation of a coffee grinding device if the hopper 300 is associated with a grinding device, not shown. In particular, the method of controlling the operation of a grinding device comprises the step of recognizing the one or more characteristics of the coffee in the hopper 300 that have changed, using the method of the present invention. Then, the method of controlling the operation of a coffee grinding device comprises the step of adjusting one or more parameters of the coffee grinding device according to the recognition signal, such as water temperature and pressure or brewing time.

According to a further embodiment, the method of monitoring at least one characteristic of a type of coffee may be used as part of a method of controlling the operation of a coffee machine if the hopper is incorporated in an espresso machine. In particular, the method of controlling the operation of a coffee machine comprises the step of recognizing the one or more characteristics of the coffee in the hopper 300 that have changed, using the method of the present invention. Then, the method of controlling the operation of a coffee machine comprises the step of adjusting one or more coffee preparation parameters according to the recognition signal, such as water temperature and pressure or brewing time.

According to a preferred embodiment, the methods of controlling the operation of a grinding device and a coffee making machine comprise the step of generating a signal indicating the change of the operating parameters of the coffee grinding device and the coffee machine when the signal representative of change exceeds a tolerance range associated with volatile organic compound concentrations as a function of the recognition signal. Preferably, these methods also comprise the step of generating a signal for maintaining the operating parameters of the coffee grinding device and the coffee machine when the change-representative signal falls within the tolerance range.

Preferably the step of controlling comprises the sub-step of cleaning each sensor 301. In particular, the control unit 306 is in signal communication with cleaning means 307 configured to clean each sensor 301. In detail, the control unit 306 is configured to periodically generate a trigger signal to trigger the cleaning means 307. More in detail, the control unit 306 triggers a sub-step of cleaning the sensor by blowing air at each sensor according to the sampling time intervals.

A further object of the present invention is an apparatus for monitoring a characteristic of a type of coffee 400 from a plurality of reference characteristics having a reference signal associated therewith.

The monitoring apparatus for monitoring a type of coffee 400 comprises the hopper 300 for storing coffee beans or ground coffee. In particular, the hopper comprises a base 300a defining a cavity 300b and a cover 300c removably associated with the base 300a. Preferably, such hopper 300 is a coffee grinding and/or dosing device.

The apparatus comprises a plurality of sensors 301 configured to sense the concentration of one or more volatile organic compounds released from the coffee held within the cavity 300b of the hopper 300 in a sampling time interval. In particular, each sensor is configured to generate a sampling signal of volatile organic compound concentrations.

The apparatus comprises the data sending and receiving unit 302 in signal communication with the plurality of sensors and configured to send the data generated by the plurality of sensors 301.

According to one embodiment, the sensors 301 are associated with the cover 300c of the hopper. Alternatively, one or more sensors 301 are associated in a housing formed in the base 300a of the hopper 300. Alternatively the sensors 301 are placed outside the hopper 301, e.g. at the base 401a and are connected to the cover 300c via a pipe, not shown. According to a preferred embodiment as shown in the figures the sensors 301 are arranged in the base 300a and on the cover 300c.

The present invention further relates to a system for monitoring a type of coffee 500 from a plurality of reference characteristics, each type of coffee being associated with a respective reference signal.

The system for monitoring at least one characteristic of the coffee 500 comprises an apparatus for recognizing a type of coffee 400 as described hereinbefore.

The system for monitoring at least one characteristic of a type of coffee 500 comprises a second data sending and receiving unit 304 in signal communication with the first data sending and receiving unit 302 and a data processing unit 303. In particular, the second data sending and receiving unit 304 is configured to receive the signals generated by the plurality of sensors 301 and preferably to send them to the data processing unit 303. The latter, for the purposes of the present invention, is configured to:
- generate a sampling signal representative of the coffee inside the hopper 300 for each sampling time interval, as a function of the volatile organic compounds that have been sensed;
- compare each sampling signal so generated to detect a change over time in one or more volatile organic compounds released from the coffee at successive sampling time intervals;
- generate a signal representative of the change over time in one or more volatile organic compounds released from the coffee when the change is sensed;
- compare the change-representative signal so generated with the reference signals associated with each reference characteristic;
- generate a characteristic-recognition signal as a function of the comparison between the representative signal that has been generated and the reference signals.

Preferably, the monitoring system 500 comprises a user interface 305 in signal communication with the data processing unit 303 and the second data sending and receiving unit 304.

In a preferred embodiment, the monitoring system 500 comprises a control unit 306 is in signal communication with the data processing unit 303 and/or with the second data sending and receiving unit 304. Preferably, the control unit 306 is of the type described above, which is configured to:
- receive the recognition signal;
- generate a signal indicating the change of the grinding and brewing parameters for the coffee inside the hopper 300. It shall be noted that the change of the grinding and brewing parameters is associated with the characteristic that has changed.

According to a preferred embodiment, the sensors 301 are associated with a hopper, a grinder and/or a grinder-doser. At least one of these (hopper, grinder and/or grinder-doser) is in signal communication, preferably via Bluetooth, with the espresso coffee machine. The latter is in signal communication, preferably in wireless or wired network communication, with the processing unit 303 arranged on site and/or remotely from the coffee machine. Preferably, the processing unit 303 comprises at least two servers in signal communication with each other. Specifically, a first server, in which the grinding and brewing parameters for the grinder (and/or the hopper and/or the grinder-doser) of the coffee machine reside, is in signal communication with the coffee machine. The first server is configured to receive/send data from/to the coffee machine and to send/receive data to/from a second server in which the algorithms for implementing the method of the present invention reside. Once the data coming from the sensors has been analyzed, the second server sends the result to the first server which in turn communicates with the coffee machine. Finally, the latter communicates with the hopper, the grinder and/or the grinder-doser. Therefore, once the characteristic recognition signal has been generated, the processing unit 303 is configured to act both on the hopper, the grinder and/or the grinder-doser and on the coffee machine. More in detail, the processing unit can adjust both the brewing parameters (temperature and pressure) on the coffee machine and the grinding parameters on the hopper, the grinder and/or the grinder-doser.

It should be noted that, in a preferred embodiment, the first and second servers are distinct and communicate via the MQTT protocol. Specifically, an explanatory example of the method of the present invention, which is carried out by the system for monitoring at least one characteristic of the coffee 500, will be given below:
1) the data acquired by the sensors are transferred from the grinder (and/or the hopper and/or the grinder-doser) to the coffee machine, for example via Bluetooth if the coffee machine is of the traditional type, i.e. with the coffee machine separated from the grinder (and/or the hopper and/or the grinder-doser). On the other hand, if the coffee machine is of the super-automatic type, i.e. with the coffee machine integrated with the grinder (and/or the hopper and/or the grinder-doser), the coffee machine, in direct signal communication with the grinder (and/or the hopper and/or the grinder-doser) equipped with the sensors, receives the data by wire;
2) once the data has been received, the coffee machine sends it to the first server (with other machine data, for example diagnostic data, recipes, etc.);
3) after receiving the data, the first server sends it to the second server in which the algorithms for implementing the method of the present invention reside, which are preferably neural network- and/or artificial intelligence-based algorithms;
4) once the algorithm has processed the acquired data, it sends the recognition signal to the first server;
5) the first server communicates with the machine about the results obtained on the recognition of the blend and possibly about how to modify the brewing and/or grinding parameters of the coffee machine and/or the grinder (and/or the hopper and/or the grinder-doser) respectively to improve the beverage brewing experience. The control unit 306 changes the machine parameters according to the signal that has been generated and the confirmation by the operator;
6) if needed, the coffee machine communicates with the grinder and/or the hopper and/or the grinder-doser) about its settings (e.g. via Bluetooth) to change the grinding parameters.

According to a preferred embodiment, the data processing unit 303 comprises a single server in signal communication with the coffee machine and in which the algorithms for implementing the method of the present invention reside.

According to a further preferred embodiment, the data processing unit 303 comprises a single server in which the algorithms for implementing the method of the present invention reside. This data processing unit 303 is in signal communication both with the coffee machine and with the hopper, the grinder and/or the grinder-doser, to act directly on each of them.

In the preferred embodiment, the system also comprises cleaning means 307 which are configured to periodically clean each sensor 301. Namely, the control unit 306 is configured to control the cleaning means 307.

According to a preferred embodiment, each sensor 301 shall be intended as a nano-sensor. Preferably, each sensor 301 has a nominal electrical resistance R0 and is configured to record a variation in the electrical resistance ΔR from the nominal resistance R0 when at least one volatile organic compound is released from the coffee in the hopper 300. Preferably, each sensor 301 comprises a chemically reactive surface 301a configured to record the electrical resistance variation in the presence of one or more volatile organic compounds. In particular, each sensor 301 comprises a heating element 301b configured to heat the chemically reactive surface 301a in a range of temperatures from 300°C to 400°C, to record the electrical resistance variation in the presence of one or more organic compounds.

In particular, the number of sensors ranges from one to four, preferably from one to eight. According to a preferred embodiment, the materials of the sensors are selected from SnO2, SnO2Au with Au, CuO and SnO2Au.

In detail, the step of sensing by of one or more sensors 301 the concentration of one or more volatile organic compounds in successive sampling time intervals comprises a conditioning sub-step in which each sensor is heated to a temperature that ranges from 300C° to 400C° depending on the type of sensor for at least 5 minutes.

The sensing step comprises a collection sub-step in order to measure for each sensor the change in electrical resistance over time. This measurement of electrical resistance variation is carried out during a predetermined time that ranges from 0.5 seconds to 10 minutes, preferably from 1 to 5 minutes, more preferably, is 3 minutes.

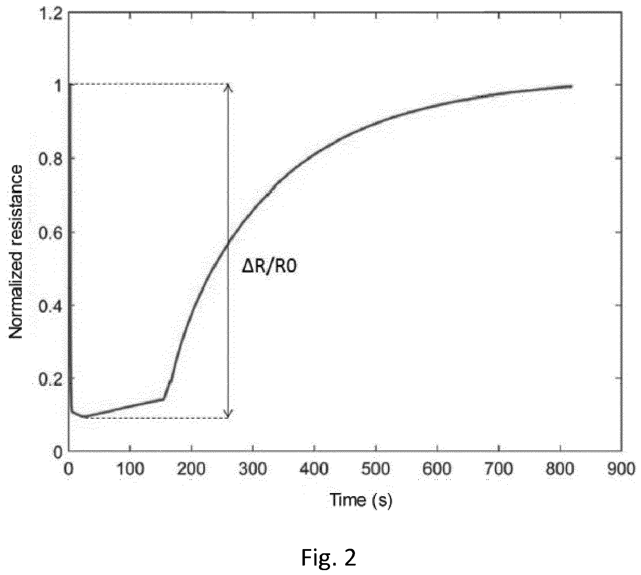

Preferably, the step of collecting, by one or more sensors 301, the concentration of one or more volatile organic compounds comprises the sub-step of normalizing each measured variation of the electrical resistance ΔRn to the nominal resistance R0 of the sensor to generate a normalized electrical resistance variation. An example of the normalized electrical resistance variation ΔRn according to the present invention is shown in Figure 2.

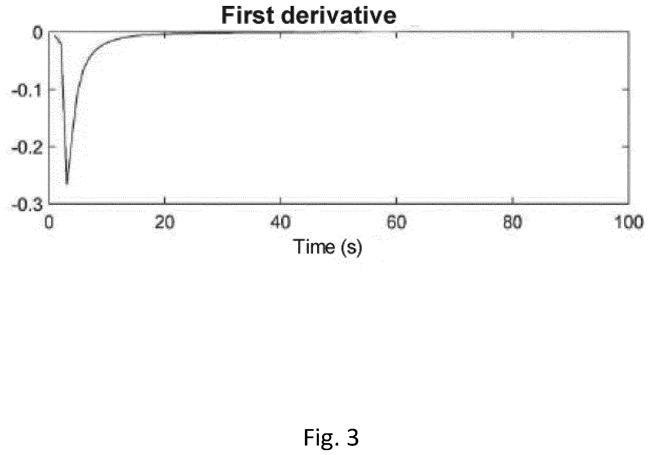

More preferably, the step of collecting by one or more sensors 301 the concentration of one or more volatile organic compounds comprises the sub-step of calculating, for at least one sensor, the first derivative dRn with respect to the time of at least one normalized variation, as shown in Figure 3. Then, the step of collecting comprises the sub-step of calculating the minimum value of the minimum Min of the calculated derivative.

The step of sensing, by one or more sensors 301, the concentration of one or more volatile organic compounds comprises the sub-step of sending at least each variation in the measured resistance ΔR to a data processing unit. In particular, each sensor 301, is in signal communication with the data processing unit 303, sends at least the variations in the measured resistance ΔR via a first data sending and receiving unit 302, optionally each sensor 301 sends the normalized variation ΔRn, the first derivative of the normalized variation dRn and the respective minimum value Min that has been calculated.

It shall be noted that optionally the sub-step of normalizing, the sub-step of calculating the first derivative and the sub-step of calculating the minimum of the first derivative are carried out by a data processing unit 303 in response to the first data sending and receiving unit 302 sending the variation in the electrical resistance as measured with time.

According to the preferred embodiment, the step of generating a coffee-sampling signal comprises the sub-step of processing each normalized variation ΔRn, the first derivative of the normalized variation dRn and the calculated value of the minimum Min.

Preferably, the coffee sampling signal for the coffee in the hopper is generated by processing the normalized variation ΔRn for each sensor in use and the minimum of the first derivative Min for at least three of the four sensors in use.

In particular, the neural network analyzes the normalized variations ΔRn and the derivatives of normalized resistance dRn, in particular the calculated values of the minimum Min, and thereby generates the sampling signal for the type of coffee.

For the purposes of the present invention, the neural network is trained with the variations of normalized electrical resistance and the minimum values of a plurality of reference coffee characteristics having a reference signal associated therewith. The neural network algorithm "learns" to recognize the characteristic of the type of coffee from the variations in the normalized electrical resistance and from the first derivatives of the variation in the normalized electrical resistance and in particular the minimum values calculated for a plurality of reference types of coffee. In detail, during the step of generating a sampling signal, the neural network algorithm receives the normalized variation ΔRn and the calculated minimum of the derivative Min and generates the coffee sampling signal.

The sensing step comprises an additional reset sub-step in which each sensor is cleaned by air injection for a time ranging from at least 10 to 30 minutes, preferably for a duration of 22 minutes.

## Claims

1. A method of monitoring at least one characteristic of a type of coffee from a plurality of reference characteristics associated with each type of coffee, each reference characteristic being associated with a respective reference signal, said method comprising the steps of:
- sensing, by one or more sensors (301), the concentration of one or more volatile organic compounds in a mixture of volatile organic compounds released from the coffee, in a sampling time interval;
- generating a coffee-representative sampling signal for each sampling time interval, as a function of the volatile organic compounds;
- comparing each sampling signal so generated to detect a change over time in one or more volatile organic compounds released from the coffee at successive sampling time intervals;
- generating a signal representative of the change over time in one or more volatile organic compounds released from the coffee when the change is sensed;
- comparing the change-representative signal so generated with the reference signals associated with each reference characteristic;
- generating a characteristic-recognition signal as a function of the comparison between the signal representative of the change over time and the reference signals.

2. Method of monitoring at least one characteristic of a type of coffee as claimed in claim 1, wherein:
- said coffee is contained in a hopper (300) of a coffee grinding and/or dosing device;
- the step of sensing senses the concentration of one or more volatile organic compounds in a mixture of volatile organic compounds released from the coffee in the hopper (300) in successive sampling time intervals over time.

3. Method of monitoring at least one characteristic of a type of coffee as claimed in claim 2, wherein the step of sensing periodically senses, in successive sampling time intervals, the concentrations of one or more volatile organic compounds in a mixture of volatile organic compounds released from the coffee contained inside the hopper (300).

4. Method of monitoring at least one characteristic of a type of coffee as claimed in claim 2 or 3, wherein the step of generating the sampling signal generates a signal representative of the concentrations of volatile organic compounds released from the coffee in a sampling time interval inside the hopper (300).

5. Method of monitoring at least one characteristic of a type of coffee as claimed in claims 2 to 4, wherein the step of comparing the sampling signals senses a change over time in one or more concentrations of volatile organic compounds sensed in the sampling time intervals inside the hopper (300).

6. Method of monitoring at least one characteristic of a type of coffee as claimed in claims 2 to 5, wherein the generation of a signal representative of a change over time generates a signal representative of the change over time in one or more concentrations of volatile organic compounds sensed inside the hopper (300).

7. Method of monitoring at least one characteristic of a type of coffee as claimed in claim 6, wherein the step of comparing the signal representative of a change over time and the reference signals associated with each reference characteristic comprises the sub-steps of:
- recognizing the volatile organic composites whose concentration has changed over time as a function of the signal representative of a change over time;
- associating at least one reference characteristic with the recognized volatile organic compounds as a function of the reference signals.

8. Method of monitoring a type of coffee as claimed in any of claims 1 to 7 wherein the step of generating a sampling signal and the step of generating a signal representative of a change over time, the step of comparing each sampling signal and the step of comparing the signal representative of a change over time with a reference signal are carried out using a neural network-based algorithm.

9. Method of monitoring a type of coffee as claimed in claim 8, wherein the neural network-based algorithm is trained to recognize one or more characteristics of a type of coffee by associating each reference characteristic with the concentration of one or more volatile organic compounds released from respective reference types of coffee.

10. Method of controlling the operation of a coffee grinding device comprising the steps of:
- sensing a change of at least one characteristic of a type of coffee contained in the hopper (300) with the method of monitoring at least one characteristic of a type of coffee as claimed in any of claims 2 to 9;
- adjusting one or more operating parameters of the coffee grinding device according to the recognition signal.

11. Method of controlling the operation of a coffee grinding device as claimed in claim 10, **characterized in that** it comprises the steps of:
- generating a signal for modifying the operating parameters of the coffee grinding device when the change-representative signal exceeds a tolerance range associated with the concentrations of volatile organic compounds as a function of the recognition signal;
- generating a signal for maintaining the operating parameters of the coffee grinding device when the change-representative signal falls within the tolerance range.

12. Method of controlling the operation of a coffee machine comprising the steps of:
- sensing a change of at least one characteristic of a type of coffee contained in the hopper (300) with the method of monitoring at least one characteristic of a type of coffee as claimed in any of claims 2 to 9;
- adjusting one or more operating parameters of the coffee machine according to the recognition signal.

13. Method of controlling the operation of a coffee machine as claimed in claim 12, **characterized in that** it comprises the steps of:
- generating a signal for modifying the operating parameters of the coffee grinding device when the change-representative signal exceeds a tolerance range associated with the concentrations of volatile organic compounds as a function of the recognition signal;
- generating a signal for maintaining the operating parameters of the coffee grinding device when the change-representative signal falls within the tolerance range.

14. An apparatus for monitoring at least one characteristic of a type of coffee (400) from a plurality of reference characteristics, each characteristic of each type of coffee being associated with a respective reference signal, said apparatus comprising:
- a hopper (300) of a grinding and/or dosing device, the hopper (300) comprising a base (300a) that defines a cavity (300b) to store the coffee and a cover (300c) that is adapted to be releasably associated with the base (300a) to close the cavity (300b); **characterized in that** it comprises:
- a plurality of sensors (301) configured to sense the presence and concentration of one or more volatile organic compounds released from the coffee stored in the hopper (300), each sensor (301) being configured to generate a signal representative of the respective volatile organic compounds in sampling time intervals.

15. A system for monitoring at least one characteristic of a type of coffee (500) from a plurality of reference characteristics, each characteristic of each type of coffee being associated with a respective reference signal, said system comprising:
- an apparatus (400) for recognizing a type of coffee from a plurality of reference types of coffee which comprises:
- a hopper (300) of a grinding and/or dosing device, the hopper (300) comprising a base (300a) that defines a cavity (300b) to store the coffee and a cover (300c) that is adapted to be releasably associable with the base (300a) to close the cavity (300b),
- a plurality of sensors (301) configured to sense the presence and concentration of one or more volatile organic compounds released from the coffee stored in the hopper (300), each sensor (301) being configured to generate a sampling signal representative of the representative volatile organic compounds that have been sensed in sampling time intervals,
- a first data sending and receiving unit (302) in signal communication with the plurality of sensors (301) and configured to send the signals generated by said plurality of sensors (301),
- a second data sending and receiving unit (304) in signal communication with the first data sending and receiving unit (302) for receiving the signals generated by said plurality of sensors (301);
- a data processing unit (303) in signal communication with the second data sending and receiving unit (304) and configured to:
- generating a sampling signal representative of the coffee inside the hopper (300) for each sampling time interval, as a function of the volatile organic compounds that have been sensed;
- comparing each sampling signal so generated to detect a change over time in one or more volatile organic compounds released from the coffee at successive sampling time intervals;
- generating a signal representative of the change over time in one or more volatile organic compounds released from the coffee when the change is sensed;
- comparing the change-representative signal so generated with the reference signals associated with each reference characteristic;
- generating a characteristic-recognition signal as a function of the comparison between the representative signal that has been generated and the reference signals.
